# EUROPEAN PATENT APPLICATION

(11) **EP 3 462 166 A2**
(43) Date of publication of application: **03.04.2019**
(21) Application number: 18194139.4
(22) Date of filing: 13.09.2018
(51) Int. Cl.: G01N 23/04, A61B 6/00

(54) **RADIATION PHASE CONTRAST IMAGING APPARATUS**

(30) Priority: 29.09.2017 JP 2017191056
(71) Applicant: Shimadzu Corporation, Kyoto 604-8511 (JP)
(72) Inventor: DOKI, Takahiro, Kyoto-shi, Kyoto 604-8511 (JP); KIMURA, Kenji, Kyoto-shi, Kyoto 604-8511 (JP); SHIRAI, Taro, Kyoto-shi, Kyoto 604-8511 (JP); SANO, Satoshi, Kyoto-shi, Kyoto 604-8511 (JP); HORIBA, Akira, Kyoto-shi, Kyoto 604-8511 (JP); MORIMOTO, Naoki, Kyoto-shi, Kyoto 604-8511 (JP); MIZUSHIMA, Hiroshi, Kyoto-shi, Kyoto 604-8445 (JP)
(74) Representative: Müller-Boré & Partner Patentanwälte PartG mbB

(57) **Abstract**

A radiation phase contrast imaging apparatus (100) is provided with an image signal generation system (3) including an X-ray source (1) and an image signal detector (2), a plurality of gratings including a first grating (4) and a second grating (5), a holder (6) for holding a plurality of gratings in a suspended manner, and a position switching mechanism (8) for switching the relative position of the plurality of gratings with respect to the image signal generation system between the retracted position and the detection position. Either one of the plurality of gratings and the image signal generation system is held by a single holder.

## Description

### BACKGROUND OF THE INVENTION

### Field of the Invention

The present invention relates to a radiation phase contrast imaging apparatus, and more particularly to a radiation phase contrast imaging apparatus capable of switching a relative position of a plurality of gratings with respect to an imaging system in accordance with an an image to be acquired.

### Background Art

Conventionally, a radiation phase contrast imaging apparatus capable of switching a relative position of a plurality of gratings with respect to an imaging system in accordance with an image to be acquired is known. Such a radiation phase contrast imaging apparatus is disclosed, for example, in US Patent No. 9,084,528. Note that an imaging system denotes an image signal generation system including an X-ray source and a detector.

In U.S. Patent No. 9,084,528, a radiation phase contrast imaging apparatus is disclosed in which an arrangement of gratings for generating an X-ray phase contrast image of a subject by fringe scanning and an arrangement of gratings for generating an image (absorption image) based on an intensity change of X-rays by a subject can be switched.

The radiation phase contrast imaging apparatus disclosed in U.S. Patent No. 9,084,528 includes an X-ray source, a source grating, a first grating, a second grating, a detector for detecting X-rays irradiated from the X-ray source which have passed through a subject, and a slider for moving the source grating, the first grating, and the second grating using a step motor.

The radiation phase contrast imaging apparatus disclosed in U.S. Patent No. 9,084,528 is configured to switch the arrangement of gratings for generating a radiation phase contrast image and the arrangement of gratings for generating an absorption image by moving a plurality of gratings by a slider.

In U.S. Patent No. 9,084,528, however, the apparatus is configured such that the slider supports gratings from below. Therefore, in cases where the apparatus is further provided with a movable subject stage for placing a subject, there is a possibility that the subject stage and the slider interfere at the time of moving the subject stage. That is, in the configuration of U.S. Patent No. 9,084,528, it is considered that the moving direction of the plurality of gratings by the slider is a horizontal direction orthogonal to the optical axis of the X-rays. In the case of, e.g., changing the imaging magnification of the subject, since the subject stage is moved in the optical axis direction of X-rays, the movement direction of the slider and the movement direction of the subject stage intersect, which causes interference between the slider and the subject stage. In order to prevent the slider from being interfered with the subject stage, the configuration is required to have redundancy in the movement direction and the movement range of the slider. Therefore, there is a problem that the device configuration becomes complicated.

The present invention has been made to solve the above problems, and aims to provide a radiation phase contrast imaging apparatus capable of suppressing complication of the apparatus configuration even in the case of moving a relative position of a plurality of gratings with respect to an image signal generation system from a retracted position to a detection position.

### SUMMARY OF THE INVENTION

In order to attain the aforementioned object, a radiation phase contrast imaging apparatus according to one aspect of the present invention includes an image signal generation system including an X-ray source and an image signal detector for detecting an image signal based on X-rays irradiated from the X-ray source; a plurality of gratings including a first grating to which the X-rays from the X-ray source are irradiated and a second grating arranged between the first grating and the image signal detector; a holder extending in an optical axis direction of the X-rays, the holder being configured to hold one of the plurality of gratings and the image signal generation system in a suspended manner; and a position switching mechanism configured to switch a relative position of the plurality of gratings with respect to the image signal generation system between a retracted position for capturing an absorption image without using the plurality of gratings and a detection position for capturing a phase contrast image using the plurality of gratings, wherein either one of the plurality of gratings and the image signal generation system is held by the holder which is single in number.

In the radiation phase contrast imaging apparatus according to one aspect of the present invention, as described above, it is provided with a single holder for holding either one of a plurality of gratings and an image signal generation system in a suspended manner and a position switching mechanism for switching the relative position of the plurality of gratings with respect to the image signal generation system between the retracted position and the detection position.

With this, the position switching mechanism can switch the relative position of the plurality of gratings with respect to the image signal generation system between the retracted position and the detection position via the single holder holding either one of the plurality of gratings and the image signal generation system in a suspended manner. As a result, it is possible to switch the relative position of the plurality of gratings with respect to the image signal generation system between the retracted position and the detection position without supporting either one of the plurality of grating and the image signal generation systems from below. With this, for example, even in cases where a subject stage or the like movable in the optical axis direction of the X-rays is provided at the bottom of the apparatus, it is possible to suppress the interference between the subject stage and the holder without adding a special structure for interference avoidance. Thereby, for example, even in cases where the relative position of the plurality of gratins with respect to the optical image generation system is moved from the retracted position to the detection position, it is possible to prevent the apparatus structure from becoming complicated.

In the radiation phase contrast imaging apparatus according to the aforementioned one aspect of the present invention, preferably, the position switching mechanism includes a moving mechanism configured to move the holder between the retracted position and the detection position by synchronously moving both ends of the holder.

Here, when capturing a phase contrast image using a plurality of gratings, imaging is performed in a state in which the relative position of the plurality of gratings with respect to the image signal generation system becomes a predetermined position. If the plurality of gratings is tilted with respect to the image signal generation system, influences, such as, e.g., degradation of the image quality of the phase contrast image to be acquired, occur. Therefore, when moving the holder from the retracted position to the detection position, the positional precision at the time of placing the holder is required. With the above configuration, it is possible to harmonize the timings at which both ends of the holder are moved. As a result, it is possible to prevent either one of the end portions of the holder from moving larger (smaller) than the other, so that it is possible to suppress inclination of the holder.

In this case, it is preferable that the moving mechanism is provided at each of both ends of the holder, and is configured to be driven by a single drive unit. With this configuration, it is possible to drive a plurality of moving mechanisms with a single drive unit, so that the driving amounts of the plurality of moving mechanisms can be equalized. As a result, movements of both ends of the holder can be easily synchronized. In addition, since it is possible to drive the plurality of moving mechanisms by a single drive unit, the device configuration can be simplified.

In the radiation phase contrast imaging apparatus according to the one aspect of the present invention, preferably, the radiation phase contrast imaging apparatus further includes a positioning portion for determining a position of the holder at the detection position by coming into contact with the holder. With this configuration, by bringing the holder into contact with the positioning portion, the position of the holder when moved from the retracted position to the detection position can be easily and accurately determined.

In this case, preferably, wherein the positioning portion is configured to determine the position of the holder in a vertical direction orthogonal to the optical axis direction by coming into contact with the holder from below the holder. With this configuration, in the detection position, the position of the holder in the vertical direction orthogonal to the optical axis can be easily determined (easily positioned).

In the radiation phase contrast imaging apparatus according to one aspect of the present invention, preferably, the positioning portion is configured to determine the position of the holder in the optical axis direction by coming into contact with the holder from the optical axis direction. With this configuration, in the detection position, the position of the holder in the optical axis direction can be easily determined (easily positioned).

In the radiation phase contrast imaging apparatus according to one aspect of the present invention, preferably, the radiation phase contrast imaging apparatus further includes a guide member having a hole part extending in a vertical direction orthogonal to the optical axis direction, wherein the holder includes a protrusion protruding in the hole part, and the hole part is provided with a first positioning portion for determining the position of the holder in the vertical direction orthogonal to the optical axis direction and a second positioning portion for determining the position of the holder in the optical axis direction. With this configuration, since the positioning in the vertical direction orthogonal to the optical axis direction and in the optical axis direction can be performed by the guide member, as compared with the case in which positioning in each direction is performed using a plurality of members, an increase in the number of parts can be suppressed. In addition, it is possible to suppress an increase in the number of parts, so that the apparatus configuration can be simplified.

In this case, preferably, the first positioning portion is provided on an inner peripheral surface of a lower end portion of the hole part, and the second positioning portion is provided on an inner peripheral surface of the hole part in the optical axis direction. With this configuration, it is possible to easily form the first positioning portion and the second positioning portion with respect to the guide member.

In the configuration further provided with a guide member having a hole part extending in a vertical direction orthogonal to the optical axis direction, preferably, the guide member is arranged so as to sandwich the holder from both sides in a horizontal direction orthogonal to the optical axis direction and configured to determine the position of the holder in the horizontal direction orthogonal to the optical axis direction. With this configuration, occurrence of positional deviation of the holder in the horizontal direction orthogonal to the optical axis direction can be suppressed.

In the radiation phase contrast imaging apparatus according to one aspect of the present invention, preferably, the position switching mechanism includes a contact part for raising and lowering the holder by coming into contact with the holder, the position switching mechanism is provided below than the positioning portion in a vertically movable manner, and the holder is configured to be supported by the positioning portion in the detection position in a state of being detached from the contact part of the position switching mechanism. With this configuration, unlike the case in which the holder is supported by a contact part of the position switching mechanism in the detection position, it is not necessary to consider the position accuracy of the contact part (position switching mechanism). As a result, there is no need to provide a mechanism for improving the position accuracy of the position switching mechanism, so that the device configuration can be simplified.

In the radiation phase contrast imaging apparatus according to one aspect of the present invention, preferably, the radiation phase contrast imaging apparatus further includes a subject position detection unit configured to detect a position of the subject, wherein when the subject is placed in an area in which the plurality of gratings is arranged at the time of placing the holder from the retracted position to the detection position, the subject position detection unit is configured to prohibit an arrangement of the holder to the detection position. With such a configuration, it is possible to avoid contact between the plurality of gratings and the subject when switching from the retracted position to the detection position.

In the radiation phase contrast imaging apparatus according to one aspect of the present invention, preferably, the radiation phase contrast imaging apparatus further includes a movable subject stage for placing a subject, the subject stage being arranged below the holder so as to face the holder. With this configuration, since the subject stage and the holder are arranged so as to face each other, it is possible to prevent the device configuration from becoming complicated in order to prevent the holder from interfering with the subject stage when moving the subject stage. As a result, when moving the subject in order to, e.g., change the imaging magnification of the subject, it is possible to easily prevent the holder from interfering with the subject stage while suppressing complication of the device configuration.

In the radiation phase contrast imaging apparatus according to one aspect of the present invention, preferably, the holder is provided with a grating position adjustment mechanism, and the grating position adjustment mechanism is configured to adjust a relative position of the plurality of gratings between gratings when the holder is placed in the detection position from the retracted position. With such a configuration, even in cases where a positional deviation occurs in the relative position the plurality of gratings among gratings as the holder moves, it can be easily adjusted. As a result, degradation of the image quality of the image to be acquired due to the positional displacement of the relative position of the plurality of gratings among gratings can be suppressed.

In the radiation phase contrast imaging apparatus according to one aspect of the present invention, preferably, the radiation phase contrast imaging apparatus further includes a focal diameter changing unit configured to change a focal diameter of the X-ray source in conjunction with switching of the holder between the retracted position and the detection position. With this configuration, it is possible to change the focal diameter of the X-ray source in conjunction with the movement of the holder. As a result, when acquiring an absorption image, it is possible to acquire an absorption image with a resolution suitable for the size (thickness) of the subject and the material.

### BRIEF DESCRIPTION OF THE DRAWINGS

FIG. 1 is a schematic view of a radiation phase contrast imaging apparatus according to an embodiment of the present invention as viewed from the X-direction.
FIG. 2A is a schematic diagram showing a detection position of a radiation phase contrast imaging apparatus according to an embodiment of the present invention.
FIG. 2B is a schematic diagram showing a retracted position of a radiation phase contrast imaging apparatus according to an embodiment of the present invention.
FIG. 3A is a schematic diagram of an arrangement of a radiation phase contrast imaging apparatus according to an embodiment of the present invention in a detection position as viewed from the X-direction.
FIG. 3B is a schematic diagram of an arrangement of a radiation phase contrast imaging apparatus according to an embodiment of the present invention in a detection position as viewed from the Z-direction.
FIG. 4A is a schematic diagram of an arrangement of a radiation phase contrast imaging apparatus according to an embodiment of the present invention in a retracted position as viewed from the X-direction.
FIG. 4B is a schematic view (B) of an arrangement of the radiation phase contrast imaging apparatus according to an embodiment of the present invention in the retracted position as viewed from the Z-direction.
FIG. 5A is an enlarged diagram showing a first guide member of a radiation phase contrast imaging apparatus according to an embodiment of the present invention.
FIG. 5B is an enlarged diagram showing a second guide member of a radiation phase contrast imaging apparatus according to an embodiment of the present invention.
FIG. 6A is an enlarged view showing a relationship between a first guide member and a protrusion of a radiation phase contrast imaging apparatus according to an embodiment of the present invention.
FIG. 6B is an enlarged diagram showing a relationship between a second guide member and a protrusion of a radiation phase contrast imaging apparatus according to an embodiment of the present invention.
FIG. 7 is a schematic diagram of a grating position adjustment mechanism of a radiation phase contrast imaging apparatus according to an embodiment of the present invention.
FIG. 8 is a schematic view of a radiation phase contrast imaging apparatus according to a first modification of one embodiment of the present invention as viewed from the X-direction.
FIG. 9 is a schematic view of a radiation phase contrast imaging apparatus according to a second modification of one embodiment of the present invention as viewed from the X-direction.
FIG. 10 is a schematic view of a radiation phase contrast imaging apparatus according to a third modification of one embodiment of the present invention as viewed from the Z-direction.
FIG. 11 is a schematic view of a radiation phase contrast imaging apparatus according to a fourth modification of one embodiment of the present invention as viewed from the Z-direction.
FIG. 12 is a schematic view of a radiation phase contrast imaging apparatus according to a fifth modification of one embodiment of the present invention as viewed from the Z-direction.
FIG. 13 is a schematic view of a radiation phase contrast imaging apparatus according to a sixth modification of one embodiment of the present invention as viewed from the X-direction.

### DESCRIPTION OF PREFERRED EMBODIMENTS

Hereinafter, embodiments of the present invention will be described with reference to the drawings.

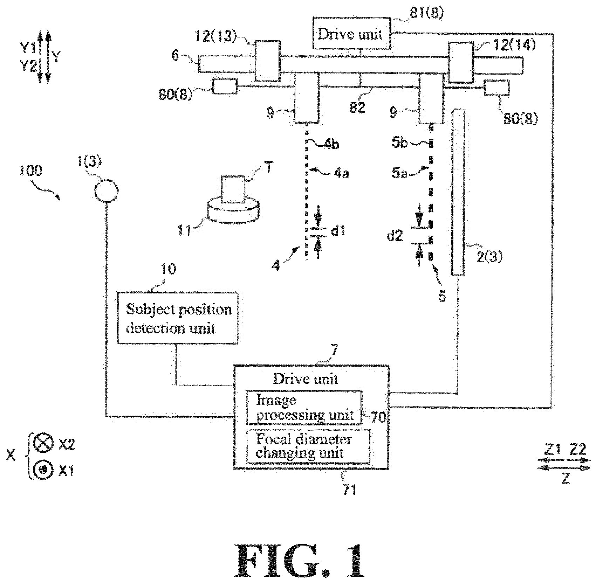

With reference to FIG. 1 to FIG. 7, the configuration of a radiation phase contrast imaging apparatus 100 according to an embodiment of the present invention will be described.

### (Configuration of Radiation Phase Contrast Imaging Apparatus)

First, with reference to FIG. 1, the configuration of the radiation phase contrast imaging apparatus 100 according to this embodiment of the present invention will be described.

As shown in FIG. 1, the radiation phase contrast imaging apparatus 100 is a device for imaging an inside of a subject T by utilizing a Talbot effect. The radiation phase contrast imaging apparatus 100 can be used for imaging an inside of a subject T as an object in, for example, a nondestructive inspection application.

FIG. 1 is a view of the radiation phase contrast imaging apparatus 100 as viewed from the X-direction. As shown in FIG. 1, the radiation phase contrast imaging apparatus 100 is provided with an image signal generation system 3 including an X-ray source 1 and an image signal detector 2, a plurality of gratings including a first grating 4 and a second grating 5, a holder 6, a control unit 7, a position switching mechanism 8, a grating position adjustment mechanism 9, a subject position detection unit 10, and a subject stage 11.

Note that in the present specification, the direction from the X-ray source 1 to the first grating 4 denotes a Z2-direction, and the direction opposite thereto denotes a Z1-direction. Further note that the left-right direction in the plane orthogonal to the Z-direction denotes an X-direction, the direction toward the back side of the paper of FIG. 1 denotes an X2-direction, and, the direction toward the front side of the paper of FIG. 1 denotes an X1-direction. Further note that the up-and-down direction in the plane orthogonal to the Z-direction denotes a Y-direction, the upward direction denotes a Y1-direction, and the downward direction denotes a Y2-direction.

The X-ray source 1 is configured to generate X-rays when a high voltage is applied and irradiate the generated X-rays in the Z2-direction.

The first grating 4 has a plurality of slits 4a and X-ray phase change portions 4b arranged at a predetermined period (pitch) d1 in the Y-direction. The slits 4a and X-ray phase change portions 4b are each formed so as to extend linearly. The slits 4a and X-ray phase change portions 4b are each formed so as to extend in parallel with each other. The first grating 4 is a so-called phase grating.

The first grating 4 is arranged between the X-ray source 1 and the second grating 5 and configured to be irradiated by the X-rays from the X-ray source 1. The first grating 4 is provided to form a self-image (not shown) of the first grating 4 by a Talbot effect. When X-rays having coherence pass through a grating in which slits are formed, an image (self-image) of the grating is formed at a position away from the grating by a predetermined distance (Talbot distance). This is called a Talbot effect.

The second grating 5 includes a plurality of X-ray transmission portions 5a and X-ray absorption portions 5b arranged in the Y-direction at a predetermined period (pitch) d2. The X-ray transmission portions 5a and the X-ray absorption portions 5b are each formed so as to extend linearly. The X-ray transmission portions 5a and the X-ray absorption portions 5b are each formed so as to extend in parallel with each other. The second grating 5 is a so-called absorption grating. The first grating 4 and the second grating 5 are gratings having different roles, but the slit 4a and the X-ray transmission portion 5a each transmit X-rays. Further, the X-ray absorption portion 5b plays a role of shielding the X-rays, and the X-ray phase change portion 4b changes the phase of the X-rays by the difference in the refractive index from the slit 4a.

The second grating 5 is arranged between the first grating 4 and the image signal detector 2, and is irradiated by the X-rays that have passed through the first grating 4. Further, the second grating 5 is arranged at a position away from the first grating 4 by the Talbot distance. The second grating 5 interferes with the self-image of the first grating 4 to form a moire fringe (not shown) on the detection surface of the image signal detector 2.

The first grating 4 and the second grating 5 are held by a single holder 6 extending in the optical axis direction (Z-direction) of the X-rays. Note that the single holder 6 may be formed not only by a single member but also by a plurality of members as long as it is configured to move integrally when moved by the position switching mechanism 8.

The image signal detector 2 is configured to detect the X-rays, convert the detected X-rays into an electric signal, and read the converted electric signal as an image signal. The image signal detector 2 is, for example, an FPD (Flat Panel Detector). The image signal detector 2 is composed of a plurality of conversion elements (not shown) and a plurality of pixel electrodes (not shown) arranged on the plurality of conversion elements. The plurality of conversion elements and pixel electrodes are arranged in an array manner in the X-direction and Y-direction at a predetermined period (pixel pitch). Further, the image signal detector 2 is configured to output the acquired image signal to the control unit 7.

The holder 6 is configured to extend in the optical axis direction (Z-direction) of the X-rays and hold a plurality of gratings including the first grating 4 and the second grating 5 in a suspended manner. The holder 6 may be formed in any shape, such as, e.g., a beam shape, a truss shape, and a rectangular tube shape. Further, the holder 6 is configured to be supported by the positioning portions 12 in the detection position. The positioning portions 12 are configured to position the holder 6 in the detection position. The configuration in which the positioning portions 12 perform positioning of the holder 6 will be described later.

The control unit 7 includes an image processing unit 70 and a focal diameter changing unit 71. The control unit 7 is configured to switch the relative position of the plurality of gratings including the first grating 4 and the second grating 5 with respect to the image signal generation system 3 between the detection position and the retracted position via the position switching mechanism 8. Further, the control unit 7 is configured to change the focal diameter of the X-ray source 1 via the focal diameter changing unit 71. The control unit 7 includes, for example, a processor, such as, e.g., a CPU (Central Processing Unit). The detection position and the retracted position will be described later.

The image processing unit 70 is configured to generate an absorption image and a phase contrast image based on the image signal output from the image signal detector 2. The image processing unit 70 includes processors, such as, e.g., a GPU (Graphics Processing Unit) and an FPGA (Field-Programmable Gate Array) configured for image processing.

The absorption image denotes an image imaged based on the difference in the absorption degree of X-rays due to the subject T. The phase contrast image denotes an image including a phase differential image and a dark field image. The phase differential image denotes an image imaged based on the phase shift of X-rays generated when X rays pass through the subject T. In addition, the dark field image denotes an image imaged based on small-angle scattering of X rays generated when X-rays pass through the subject T.

The focal diameter changing unit 71 is configured to change the focal diameter of the X-ray source 1 in conjunction with the switching between the retracted position and the detection position of the holder 6 based on the signal from the control unit 7. Specifically, the focal diameter changing unit 71 is configured to change the focal diameter of the X-ray source 1 by changing the focus control of the X-ray source 1.

The position switching mechanism 8 includes a moving mechanism 80 and a drive unit 81. The position switching mechanism 8 is configured to switch the relative position of the plurality of gratings including the first grating 4 and the second grating 5 with respect to the image signal generation system 3 between the retracted position and the detection position via the holder 6 based on the signal from the control unit 7.

The retracted position denotes a relative position of the plurality of gratings with respect to the image signal generation system 3 when capturing an absorption image without using a plurality of gratings. The detection position denotes a relative position of the plurality of gratings with respect to the image signal generation system 3 when capturing the phase contrast image using the plurality of gratings.

Specifically, the control unit 7 is configured to drive the drive unit 81. The position switching mechanism 8 is configured to move a plurality of gratings including the first grating 4 and the second grating 5 to the detection position and the retracted position by the moving mechanism 80 to which power is transmitted from the drive unit 81. The detailed configuration of the position switching mechanism 8 will be described later.

The grating position adjustment mechanism 9 is configured to adjust the relative position of the plurality of gratings among gratings when the holder 6 is placed in the detection position. The grating position adjustment mechanism 9 is provided on the Y2-direction side of the holder 6. Further, the grating position adjustment mechanism 9 is configured to hold the gratings. That is, the plurality of gratings are configured to be held by the holder 6 via the grating position adjustment mechanism 9. The detailed configuration of the grating position adjustment mechanism 9 will be described later.

The subject position detection unit 10 is configured to detect the position of subject T. Further, the subject position detection unit 10 is configured to output a signal to the control unit 7 when the subject T is placed in an area in which the plurality of gratings is arranged at the time of arranging the holder 6 from the retracted position to the detection position. The control unit 7 is configured to prohibit the placement of the holder 6 in the detection position based on the signal from the subject position detection unit 10. The subject position detection unit 10 includes, for example, an optical sensor such as an infrared sensor, an image sensor (camera), and the like. The subject position detection unit 10 may be arbitrarily configured as long as it can detect the position of the subject T.

The subject stage 11 is configured so that the subject T is arranged. The subject stage 11 is configured to move the subject T in the optical axis direction (Z-direction) based on the signal from the control unit 7 at the time of changing the imaging magnification of the subject T, for example, in the case of imaging the subject T in an enlarged manner or in a reduced manner. Further, the subject stage 11 is arranged so as to face the holder 6 below the holder 6 (Y2-direction). The subject stage 11 is, for example, a platform for placing the subject T, and includes, e.g., a motor for moving the platform.

### (Detection Position and Retracted Position)

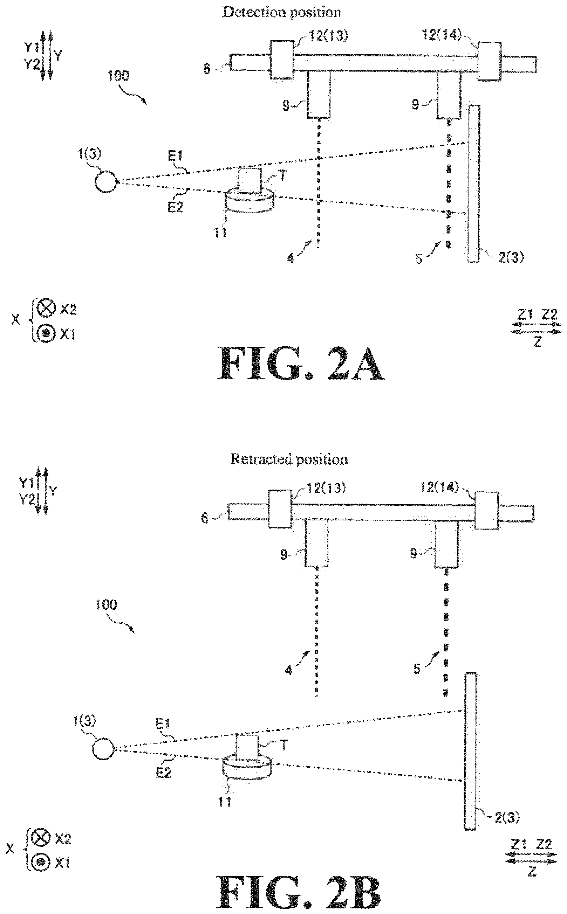

Next, with reference to FIG. 2, the detection position and the retracted position of the radiation phase contrast imaging apparatus 100 according to an embodiment of the present invention will be described.

The detection position in the present embodiment denotes a relative position for placing the holder 6 so that the holder 6 is arranged within the irradiation range in which the first grating 4 and the second grating 5 are irradiated by the X-rays emitted from the X-ray source 1 as shown in FIG. 2A. Strictly speaking, the relative position where the plurality of gratings is arranged so as to be within the irradiation range of the X-rays that have passed through the subject T is set as the detection position. In the example shown in FIG. 2A, the range determined by the straight line E1 and the straight line E2 in the irradiation range of the X-rays detected by the image signal detector 2 is the range irradiated with the X-rays that have passed through the subject T.

In addition, the retracted position is a relative position where the holder 6 is arranged so that the first grating 4 and the second grating 5 are arranged outside the irradiation range of the X-rays emitted from the X-ray source 1 as shown in FIG. 2B. Strictly speaking, it is assumed that a relative position where the plurality of gratings are arranged in the range irradiated with X-rays which have not passed through the subject T is included in the retracted position. That is to say, in cases where the plurality of gratings is arranged so as to fall within the range to be irradiated with X-rays which have not passed through the subject T among the X-rays reaching the image signal detector 2, it may be regarded as a retracted position. In the example shown in FIG. 2B, the range other than being determined by the straight line E1 and the straight line E2 in the irradiation range of the X-rays detected by the image signal detector 2 is the range irradiated with the X-rays which have not been passed through the subject T.

In this embodiment, the radiation phase contrast imaging apparatus 100 is configured to capture a phase contrast image using the plurality of gratings held by the holder 6 in the detection position. Further, the radiation phase contrast imaging apparatus 100 is configured to capture the absorption image without using the plurality of gratings held by the holder 6 in the retracted position. As shown in FIG. 2, the radiation phase contrast imaging apparatus 100 is configured to switch between the retracted position and the detection position by moving the holder 6 in the up-and-down direction (Y-direction).

### (Configuration of Position Switching Mechanism)

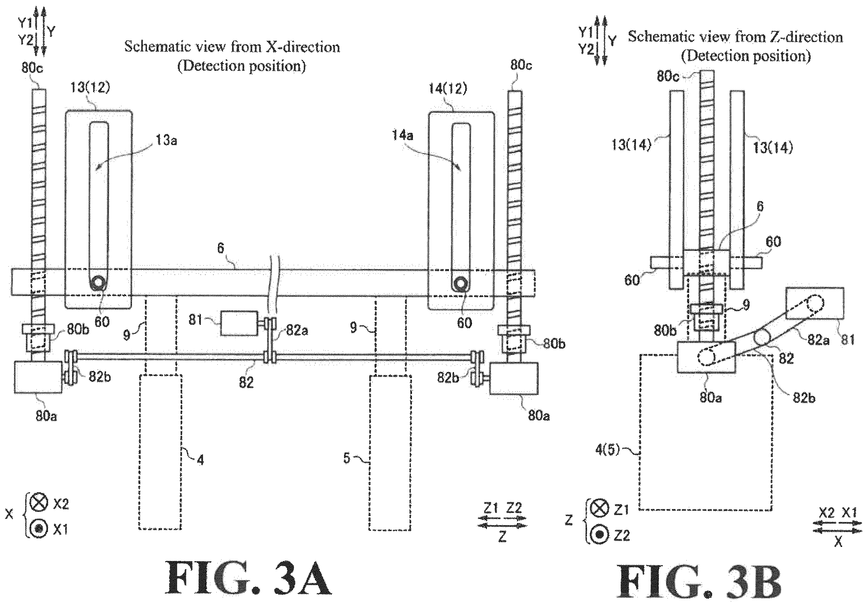

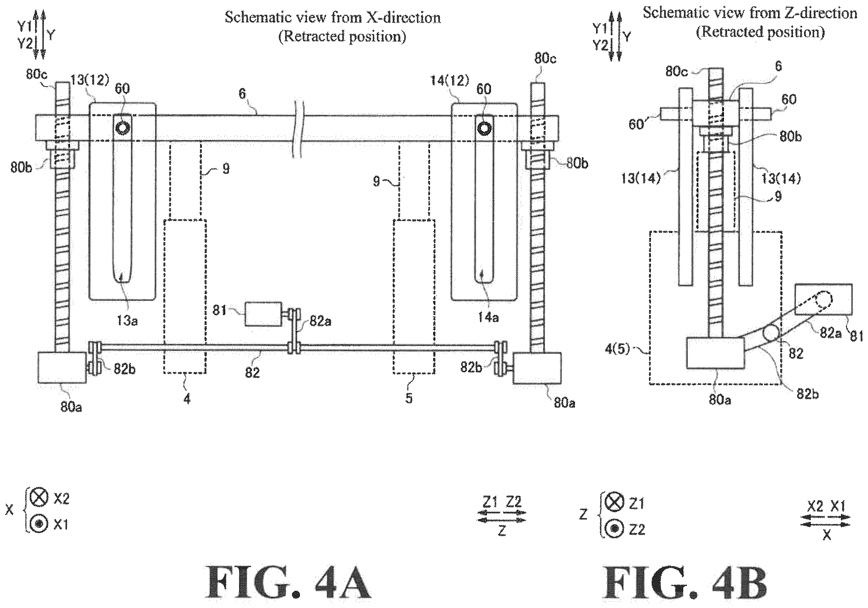

Next, with reference to FIG. 3 and FIG. 4, the configuration of the position switching mechanism 8 in this embodiment will be described.

FIG. 3 is a schematic diagram showing the arrangement of the holder 6 in the detection position and the position switching mechanism 8. As shown in FIG. 3A, a moving mechanism 80 is disposed at both ends of the holder 6. The moving mechanism 80 is configured to move the holder 6 between the retracted position and the detection position by synchronously moving both ends of the holder 6. Further, the position switching mechanism 8 includes a single drive unit 81. The position switching mechanism 8 includes a drive shaft 82 configured to transmit the power from the drive unit 81 to the moving mechanism 80. The drive shaft 82 is connected to the drive unit 81 by a belt 82a. The drive shaft 82 is connected to the moving mechanism 80 by a belt 82b. As shown in FIG. 2A, the position switching mechanism 8 is configured such that the power from the drive unit 81 is transmitted to the drive shaft 82 via the belt 82a. In the moving mechanism 80, the power is transmitted from the drive shaft 82 via the belt 82b.

As shown in FIG. 3A, the moving mechanism 80 includes a power input unit 80a to which the power from the drive unit 81 is input, a contact part 80b that moves the holder 6 up and down by contacting the holder 6, and a shaft unit 80c that moves the contact part 80b up and down. The moving mechanism 80 is a linear motion mechanism for moving the holder 6 up and down. The moving mechanism 80 is, for example, a ball screw mechanism. That is, the shaft unit 80c is a ball screw shaft extending vertically, and the contact part 80b is a ball nut screwed to the shaft unit 80c. Note that it is sufficient that the moving mechanism 80 moves the holder 6 up and down, so the moving mechanism may be any mechanism, such as, e.g., a belt pulley mechanism and a rack and pinion mechanism.

As shown in FIG. 3A, the positioning portion 12 includes a first guide member 13 and a second guide member 14. The first guide member 13 and the second guide member 14 are provided near both ends of the holder 6. The position switching mechanism 8 is provided so as to be movable up and down below than the positioning portion 12 (the first guide member 13 and the second guide member 14). In the detection position, the holder 6 is configured to be supported by the positioning portion 12 (the first guide member 13 and the second guide member 14) in a state in which the surface of the holder 6 on the Y2-direction side is away from the surface of the contact part 80b of the position switching mechanism 8 on the Y1-direction side. The structure of the first guide member 13 and the second guide member 14 will be described later.

As shown in FIG. 3B, the first guide member 13 (second guide member 14) is arranged so as to sandwich the holder 6 from both sides in the horizontal direction (X-direction) orthogonal to the optical axis direction. The pair of first guide members 13 (second guide members 14) are configured to determine the position of the holder 6 in the horizontal direction orthogonal to the optical axis direction.

Also, as shown in FIG. 3, the holder 6 includes a protrusion 60 protruding into the hole part 13a of the first guide member 13. The first guide member 13 is configured to perform positioning of the holder 6 by coming into contact with the protrusion 60. The configuration in which the first guide member 13 positions the holder 6 will be described later.

FIG. 4 is a schematic diagram showing the arrangement of the holder 6, the first guide member 13, and the position switching mechanism 8 in the retracted position. As shown in FIG. 4A, the position switching mechanism 8 is configured to arrange the holder 6 in the retracted position by bringing the surface of the contact part 80b of the position switching mechanism 8 on the Y1-direction side into contact with the surface of the holder 6 on the Y2-direction side from the Y1-direction.

In this embodiment, the position switching mechanism 8 is configured to move the holder 6 between the retracted position and the detection position by synchronously moving both ends of the holder 6. Specifically, the position switching mechanism 8 is configured to drive the moving mechanisms 80 respectively provided at both ends of the holder 6 from a single drive unit 81 via the drive shaft 82. Therefore, the position switching mechanism 8 can synchronously move both ends of the holder 6. The holder 6 moves in parallel in the vertical direction (Y-direction) while maintaining the horizontal state. The protrusions 60 are guided in the hole part 13a of the first guide member 13 and the hole part 14a of the second guide member 14.

### (Positioning Portion)

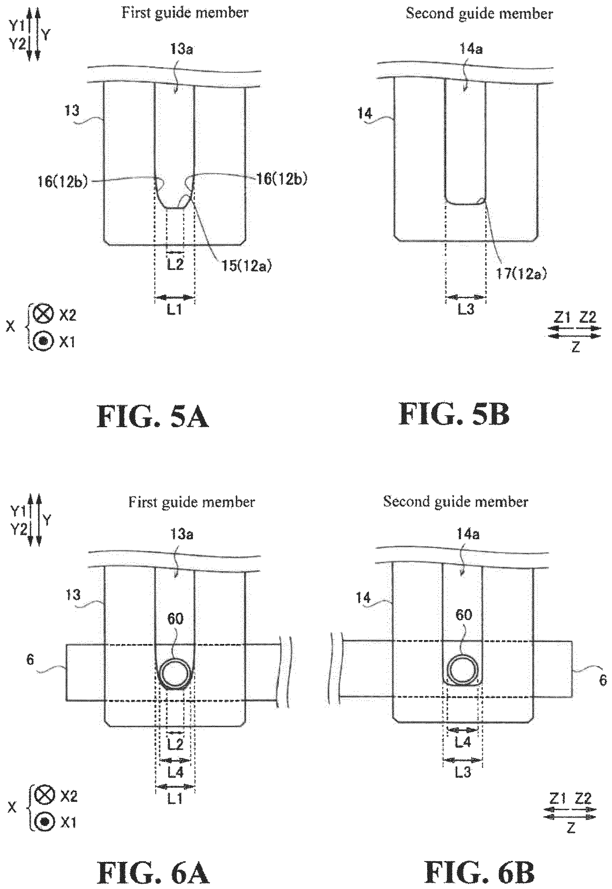

Next, with reference to FIG. 5, the structure of the positioning portion 12 in this embodiment will be described.

FIG. 5A is a schematic diagram of the first guide member 13 provided with a first positioning portion 12a and a second positioning portion 12b. FIG. 5B is a schematic diagram of the second guide member 14 not provided with the second positioning portion 12b.

As shown in FIG. 5A, the first guide member 13 has a hole part 13a extending in a vertical direction (Y-direction) orthogonal to the optical axis direction. The hole part 13a is formed so that the length L1 (the inner dimension of the hole part 13a in the optical axis direction (Z-direction)) between the opposed inner peripheral surfaces 16 of the hole part 13a is larger than the length L2 of the inner peripheral surface 15 at the lower end of the hole part 13a in the Z-direction. That is, the hole part 13a is formed in a tapered shape in which the length in the optical axis direction (Z-direction) becomes shorter toward the lower end part (the end part in the Y2-direction). Further, the inner peripheral surface 15 at the lower end portion of the first guide member 13 is configured to support the protrusion 60 from below (Y2-direction).

As shown in FIG. 5B, the second guide member 14 has a hole part 14a extending in a vertical direction (Y-direction) orthogonal to the optical axis direction. The hole part 14a is configured so that the length L3 of the inner peripheral surface 17 in the Z-direction is substantially the same. Further, the inner peripheral surface 17 of the lower end portion of the second guide member 14 is configured to support the protrusion 60 from below (Y2-direction).

As shown in FIG. 6A, the protrusion 60 is configured to protrude into the hole part 13a of the first guide member 13. The length L4 of the protrusion 60 in the optical axis direction (Z-direction) is formed so as to be shorter than the length L1 of the hole part 13a in the optical axis direction (Z-direction). The length L4 of the protrusion 60 in the optical axis direction (Z-direction) is formed to be longer than the length L2 of the inner peripheral surface 15 at the lower end of the hole part 13a in the Z-direction.

In the detection position, the hole part 13a is formed at an inclination angle such that the inner peripheral surface 16 of the hole part 13a in the optical axial direction (Z-direction) comes into contact with both sides of the protrusion 60 in the Z-direction when the inner peripheral surface 15 of the lower end comes into contact with the protrusion 60 protruding into the hole part 13a from below (Y2-direction). With this, the position of the holder 6 in the optical axis direction (Z-direction) is determined by the inner peripheral surface 16 of the hole part 13a in the optical axis direction (Z-direction). The inner peripheral surface 15 of the lower end of the hole part 13a is an example of the "first positioning portion" recited in claims. The inner peripheral surface 16 of the hole part 13a in the optical axis direction (Z-direction) is an example of the "second positioning portion" recited in claims.

FIG. 6B is a schematic diagram of the second guide member 14 not provided with the second positioning portion 12b. As shown in FIG. 6B, the length L3 of the hole part 14a of the second guide member 14 in the optical axis direction (Z-direction) is formed so as to be larger than the length L4 of the protrusion 60 in the optical axis direction (Z-direction). In the detection position, when the second guide member 14 comes into contact with the protrusion 60 from the lower side (Y2-direction), it comes into contact with the inner peripheral surface 17 of the lower end portion of the second guide member 14. Therefore, the position of the holder 6 in the optical axis direction (Z-direction) is determined by the first guide member 13 and the second guide member 14. On the other hand, since the second guide member 14 does not have the second positioning portion 12b, in the second guide member 14, the protrusion 60 is configured to be movable in the optical axis direction (Z-direction). Therefore, the positioning of the holder 6 in the optical axis direction (Z-direction) is performed by the first guide member 13. Each of the first guide member 13 and the second guide member 14 is configured so as to be able to adjust the position in the vertical direction (Y-direction) orthogonal to the optical axis direction. Therefore, by adjusting the positions of the first guide member 13 and the second guide member 14 in the vertical direction (Y-direction) orthogonal to the optical axis direction, it is possible to hold the holder 6 horizontally in the detection position. Further, by the pair of first guide members 13, positioning in the horizontal direction (X-direction) orthogonal to the optical axis direction and the optical axis direction (Z-direction) can be performed. Further, it is possible to perform positioning of the holder 6 in the vertical direction (Y-direction) orthogonal to the optical axis direction by the pair of first guide members 13 and the pair of second guide members 14. Note that the inner peripheral surface 17 of the lower end of the hole part 14a is an example of the "first positioning portion" recited in claims.

### (Grating Position Adjustment Mechanism)

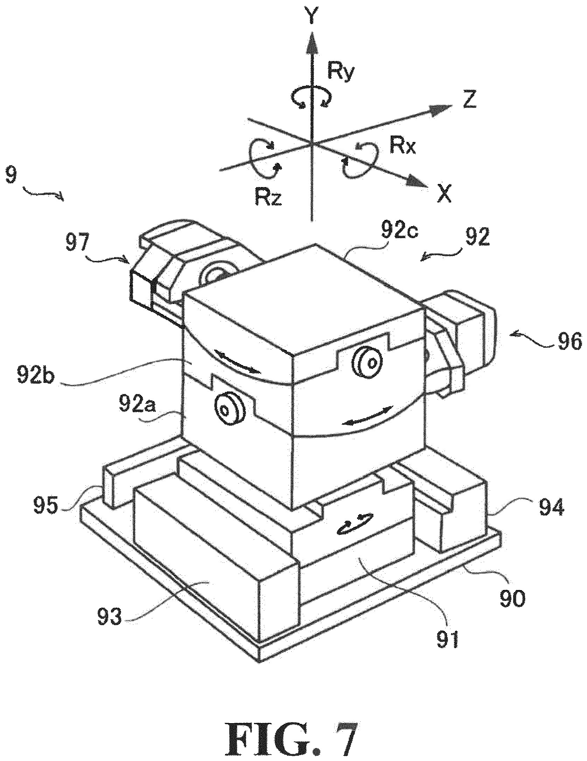

Next, the configuration of the grating position adjustment mechanism 9 will be described with reference to FIG. 7.

The grating position adjustment mechanism 9 is configured to adjust the relative position of each grating among gratings when the holder 6 is placed in the detection position by the position switching mechanism 8. Further, the grating position adjustment mechanism 9 is configured to perform scanning of the second grating 5 based on the signal from the control unit 7 at the time of imaging.

As shown in FIG. 7, the grating position adjustment mechanism 9 is configured by stacking a plurality of positioning mechanisms for moving the plurality of gratings in different directions. Specifically, the grating position adjustment mechanism 9 is configured by stacking a plurality of positioning mechanisms in the Y-direction. More specifically, the grating position adjustment mechanism 9 includes a base unit 90, a stage support unit 91, a stage 92 for mounting a grating, a first drive unit 93, a second drive unit 94, a third drive unit 95, a fourth drive unit 96, and a fifth drive unit 97. Each of the first to fifth drive units includes, for example, a motor or the like. The stage 92 is composed of a connecting portion 92a, an about X-axis direction rotating unit 92b, and an about Z-axis direction rotating unit 92c. Further, the stage 92 is configured as a grating holder for holding each grating.

The first drive unit 93, the second drive unit 94, and the third drive unit 95 are each provided on the upper surface of the base unit 90. The first drive unit 93 is configured to reciprocate the stage support unit 91 in the X-direction. Further, the second drive unit 94 is configured to rotate the stage support unit 91 about the Y-axis direction. The third drive unit 95 is configured to reciprocate the stage support unit 91 in the Z-direction. The stage support unit 91 is connected to the connecting portion 92a of the stage 92. As the stage support unit 91 moves, the stage 92 also moves.

Further, the fourth drive unit 96 is configured to reciprocate the about X-axis direction rotating unit 92b in the Z-direction. The about X-axis direction rotating unit 92b is formed so that the bottom surface thereof is formed into a convex curved surface toward the connecting portion 92a, and is configured to rotate the stage 92 about the central axis of the X-direction by being reciprocated in the Z-direction. Further, the fifth drive unit 97 is configured to reciprocate the about Z-axis direction rotating unit 92c in the X-direction. The about Z-axis direction rotating unit 92c is formed so that the bottom surface thereof is formed into a convex curved surface toward the about X-axis direction rotating unit 92b, and is configured to rotate the stage 92 about the central axis of the Z-direction by being reciprocated in the X-direction.

Therefore, the grating position adjustment mechanism 9 is configured so that the grating can be adjusted in the X-direction by the first drive unit 93. Further, the grating position adjustment mechanism 9 is configured so that the grating can be adjusted in the rotation direction about the Y-axis direction (Ry-direction) by the second drive unit 94. Further, the grating position adjustment mechanism 9 is configured so that the grating can be adjusted in the Z-direction by the third drive unit 95. Further, the grating position adjustment mechanism 9 is configured so that the grating can be adjusted in the rotation direction about the X-axis direction (Rx-direction) by the fourth drive unit 96. Further, the grating position adjustment mechanism 9 is configured so that the grating can be adjusted in the rotation direction about the Z-axis direction (Rz-direction) by the fifth drive unit 97. The movable range in each axis direction is, for example, several mm (millimeters). Further, the rotatable angle in the rotational direction Rx about the X-direction axis, the rotational direction Ry about the Y-direction axis, and the rotational direction Rz about the Z-direction axis are each, for example, several degrees.

When capturing a phase contrast image using a plurality of gratings, imaging is performed by arranging the holder 6 in the detection position. Therefore, the grating position adjustment mechanism 9 is configured to adjust the relative position among gratings of a plurality of gratings by adjusting the position in each axis direction and around each axis when the holder 6 is switched to the detection position. When the holder 6 is arranged in the retracted position in order to capture the absorption image without using a plurality of gratings, the plurality of gratings is not used for imaging, so the position adjustment of the plurality of gratings is not performed.

### (Effects of Embodiment)

In this embodiment, the following effects can be obtained.

In the first embodiment, as described above, the radiation phase contrast imaging apparatus 100 is provided with an image signal generation system 3 including the X-ray source 1 and the image signal detector 2 for detecting an image signal based on X-rays irradiated from the X-ray source 1, a plurality of gratings including the first grating 4 to which X-rays from the X-ray source 1 are irradiated and the second grating 5 disposed between the first grating 4 and the image signal detector 2, the holder 6 extending in the optical axis direction (Z-direction) of the X-ray and holding a plurality of gratings in a suspended manner, and the position switching mechanism 8 configured to switch the relative position of the plurality of gratings with respect to the image signal generation system 3 between a retracted position in which an absorption image is captured without using the plurality of gratings and a detection position in which a phase contrast image is captured using the plurality of gratings. The plurality of gratings is held by the single holder 6. With this, the position switching mechanism 8 can switch the relative position of the plurality of gratings with respect to the image signal generation system 3 between the retracted position and the detection position via the single holder 6 holding the plurality of gratings in a suspended manner. As a result, it is possible to switch the relative position of the plurality of gratings with respect to the image signal generation system 3 between the retracted position and the detection position without supporting the plurality of gratings from below. With this, for example, even in cases where a subject stage 11 or the like movable in the optical axis direction (Z-direction) of the X-rays is provided at the bottom of the apparatus, it is possible to suppress the interference between the subject stage 11 and the holder 6 without adding a special structure for interference avoidance. Thereby, for example, even in cases where the relative position of the plurality of gratins with respect to the image signal generation system 3 is moved from the retracted position to the detection position, it is possible to prevent the apparatus structure from becoming complicated.

Further, in this embodiment, as described above, the position switching mechanism 8 is provided with the moving mechanism 80 configured to move the holder 6 to the retracted position and the detection position by synchronously moving both ends of the holder 6. Here, when capturing a phase contrast image using a plurality of gratings, imaging is performed in a state in which the relative position of the plurality of gratings with respect to the image signal generation system 3 becomes a predetermined position. If the plurality of gratings is tilted with respect to the image signal generation system 3, influences, such as, e.g., degradation of the image quality of the phase contrast image to be acquired, occur. Therefore, when moving the holder 6 from the retracted position to the detection position, the positional precision at the time of placing the holder 6 is required. By synchronously moving both ends of the holder 6, it becomes possible to match the timing at which both ends of the holder 6 are moved. As a result, it is possible to prevent either one of the end portions of the holder 6 from moving larger (smaller) than the other, so that it is possible to suppress inclination of the holder 6.

Further, in this embodiment, as described above, the moving mechanism 80 is provided at each end of the holder 6, and is configured to be driven by a single drive unit 81. With this, it is possible to drive the plurality of moving mechanisms 80 with a single drive unit 81, so that the driving amounts of the plurality of moving mechanisms can be equalized. As a result, movements of both ends of the holder 6 can be easily synchronized. Further, since it is possible to drive the plurality of moving mechanisms 80 by the single drive unit 81, the device configuration can be simplified.

Further, in this embodiment, as described above, the radiation phase contrast imaging apparatus 100 is further provided with the positioning portion 12 for determining the position of the holder 6 in the detection position by coming into contact with the holder 6. With this configuration, by bringing the holder 6 into contact with the positioning portion 12, the position of the holder 6 when moved from the retracted position to the detection position can be easily and accurately determined.

Further, in this embodiment, as described above, the positioning portion 12 is configured to determine the position of the holder 6 in the vertical direction (Y-direction) orthogonal to the optical axis direction by contacting from below (Y1-direction) the holder 6. With this configuration, in the detection position, the position of the holder 6 in the vertical direction (Y-direction) orthogonal to the optical axis can be easily determined (easily positioned).

Further, in this embodiment, as described above, the image processing unit 70 is configured to determine the position of the holder 6 in the optical axis direction (Z-direction) by bringing the positioning portion 12 into contact with the holder 6 from the optical axis direction (Z-direction). With this configuration, in the detection position, the position of the holder 6 in the optical axis direction (Z-direction) can be easily determined (easily positioned).

Further, in this embodiment, as described above, the radiation phase contrast imaging apparatus 100 is further provided with the first guide member 13 having the hole part 13a extending in the vertical direction (Y-direction) orthogonal to the optical axis direction. The holder 6 includes the protrusion 60 protruding into the hole part 13a. The hole part 13a is provided with the first positioning portion 12a for determining a position of the holder 6 in a vertical direction (Y-direction) orthogonal to the optical axis direction and the second positioning portion 12b for determining a position of the holder 6 in the optical axis direction (Z-direction). With this configuration, since the positioning in the vertical direction (Y-direction) orthogonal to the optical direction and in the optical axis direction (Z-direction) can be performed by the first guide member 13, as compared with the case in which positioning in each direction is performed using a plurality of members, an increase in the number of parts can be suppressed. In addition, it is possible to suppress an increase in the number of parts, so that the apparatus configuration can be simplified.

Further, in this embodiment, as described above, the first positioning portion 12a is provided on the inner peripheral surface 15 of the lower end of the hole part 13a and the second positioning portion 12b is provided on the inner peripheral surface 16 in the optical axis direction (Z-direction) of the hole part 13a. With this configuration, it is possible to easily form the first positioning portion 12a and the second positioning portion 12b with respect to the first guide member 13.

Further, in this embodiment, as described above, the first guide member 13 is arranged so as to sandwich the holder 6 from both sides in a horizontal direction (X-direction) orthogonal to the optical axis direction and configured to determine the position of the holder 6 in the horizontal direction (X-direction) orthogonal to the optical axis direction. With this configuration, occurrence of positional deviation of the holder 6 in the horizontal direction (X-direction) orthogonal to the optical axis direction can be suppressed.

Further, in this embodiment, as described above, the position switching mechanism 8 includes a contact part 80b for raising and lowering the holder 6 by abutting against the holder 6, the position switching mechanism 8 is provided below the positioning portion 12 in a vertically movable manner, and the holder 6 is configured to be supported by the positioning portion 12 at the detection position in a state of being detached from the contact part 80b of the position switching mechanism 8. With this configuration, unlike the case in which the holder 6 is supported by a contact part 80b of the position switching mechanism 8 in the detection position, it is not necessary to consider the position accuracy of the contact part 80b (position switching mechanism). As a result, there is no need to provide a mechanism for improving the position accuracy of the position switching mechanism 8, so that the device configuration can be simplified.

Further, in this embodiment, as described above, the subject position detection unit 10 for detecting the position of the subject T is further provided, and the subject position detection unit 10 is configured such that when the holder 6 is placed from the retracted position to the detection position, placement of the holder 6 to the detection position is prohibited when the subject T is placed in an area in which a plurality of gratings are arranged. With this, it is possible to prevent contact between the plurality of gratings and the subject T when switching from the retracted position to the detection position.

Further, in this embodiment, as described above, the radiation phase contrast imaging apparatus 100 is further provided with the subject stage 11 disposed below the holder 6 so as to face the holder 6 and configured to place the subject T. With this configuration, since the subject stage 11 and the holder 6 are arranged so as to face each other, it is possible to prevent the device configuration from becoming complicated in order to prevent the holder 6 from interfering with the subject stage 11 when moving the subject stage 11. As a result, when moving the subject T in order to, e.g., change the imaging magnification of the subject T, it is possible to easily prevent the holder 6 from interfering with the subject stage 11 while suppressing complication of the device configuration.

Further, in this embodiment, as described above, the holder 6 is provided with a the grating position adjustment mechanism 9, and the grating position adjustment mechanism 9 is configured to adjust the relative position of the plurality of gratings among gratings when the holder is placed at the detection position from the retracted position. With this configuration, even in cases where a positional deviation occurs in the relative position the plurality of gratings among gratings as the holder moves, it can be easily adjusted. As a result, degradation of the image quality of the image to be acquired due to the positional displacement of the relative position of the plurality of gratings among gratings can be suppressed.

Further, in this embodiment, as described above, it further includes the focal diameter changing unit 71 for changing the focal diameter of the X-ray source 1 in conjunction with the switching between the retracted position and the detection position of the holder 6. With this configuration, it is possible to change the focal diameter of the X-ray source 1 in conjunction with the movement of the holder 6. As a result, when acquiring an absorption image, it is possible to acquire an absorption image with a resolution suitable for the size (thickness) of the subject T and the material.

### (Modifications)

It should be understood that the embodiments disclosed here are examples in all respects and are not restrictive. The scope of the present invention is shown by the scope of the claims rather than the descriptions of the embodiments described above, and includes all changes (modifications) within the meaning of equivalent and the scope of claims.

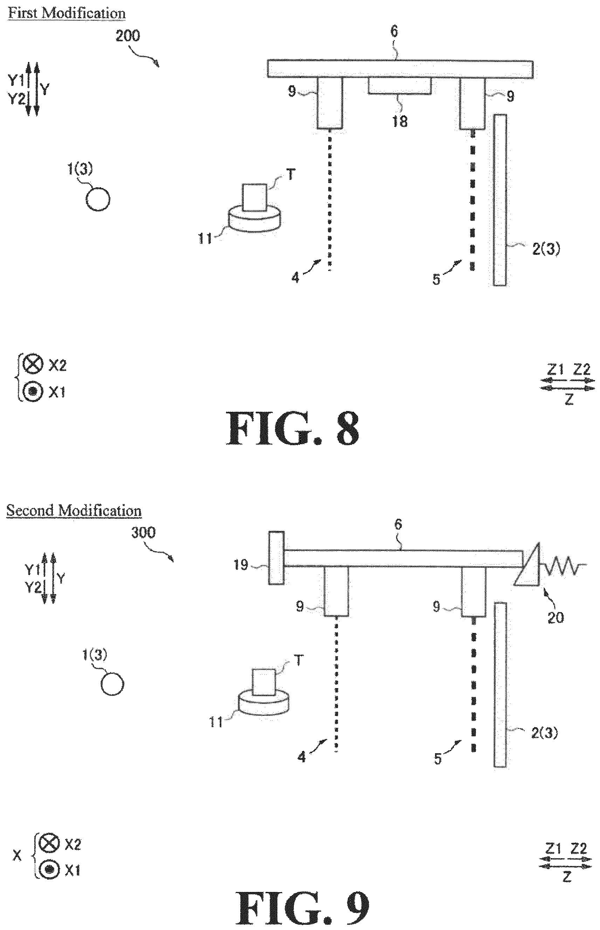

For example, in the aforementioned embodiment, although an example is shown in which the positioning of the holder 6 in the vertical direction (Y-direction) orthogonal to the optical axis direction is performed by the first positioning portion 12a of the first guide member 13, the present invention is not limited thereto. For example, like in the radiation phase contrast imaging apparatus 200 shown in FIG. 8, it may be configured such that the first contact part material 18 coming into contact with the holder 6 from the lower side (Y1-direction) is provided so as to perform positioning of the holder 6 in the vertical direction (Y-direction) orthogonal to the optical axis direction by the first contact part material 18. Note that the first contact part material 18 is an example of the "positioning portion" recited in claims.

Further, in the aforementioned embodiment, although an example is shown in which the positioning of the holder 6 in the vertical direction (Z-direction) orthogonal to the optical axis direction is performed by the second positioning portion 12b of the first guide member 13, the present invention is not limited thereto. For example, like in the radiation phase contrast imaging apparatus 300 shown in FIG. 9, it may be configured to provide a second contact part member 19 which comes into contact with one end of the holder 6 in the optical axis direction (Z-direction) and a first urging member 20 which urges the holder 6 from the other end of the holder 6 in the optical axial direction (Z-direction) and positioning of the holder 6 is performed in the optical axis direction (Z-direction). Note that the second contact part member 19 is an example of the "positioning portion" recited in claims.

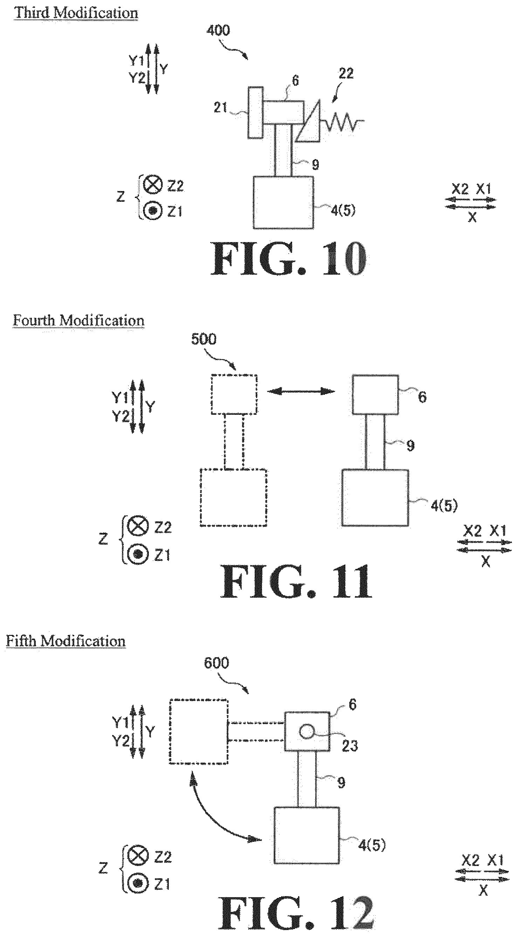

Further, in the aforementioned embodiment, although an example is shown in which the positioning of the holder 6 in the horizontal direction (X-direction) orthogonal to the optical axis direction is performed by the first guide member 13, the present invention is not limited thereto. For example, like in the radiation phase contrast imaging apparatus 400 shown in FIG. 10, it may be configured such that a third contact part member 21 which comes into contact with on one end of the holder 6 in the X-direction and a second urging member 22 for urging the holder 6 from the other end of the holder 6 in the X-direction are provided and positioning of the holder 6 in the horizontal direction (X-direction) orthogonal to the optical axis direction is performed. Note that the third contact part member 21 is an example of the "positioning portion" recited in claims.

Further, in the aforementioned embodiment, although an example is shown in which the retracted position and the detection position are switched by moving the holder 6 in the vertical direction (Y-direction) orthogonal to the optical axis direction, the present invention is not limited to this. For example, like in the radiation phase contrast imaging apparatus 500 shown in FIG. 11, it may be configured to switch the retracted position (see the two-dot chain line) and the detection position by moving the holder 6 in the horizontal direction (X-direction) orthogonal to the optical axis direction. Further, it may be configured to move the holder 6 in a direction slightly inclined from a horizontal direction (X-direction) orthogonal to the optical axis direction. With this configuration, even in cases where there is a possibility that the holder 6 slightly repels by the positioning portion 12 when it comes into contact with the positioning portion 12, the holder 6 is urged in the direction of gravity due to its own weight, there becomes no need to continuously drive the drive unit 81 to suppress the repulsion of the holder 6. Occurrence of a load on the drive unit 81 can be suppressed.

Further, in the aforementioned embodiment, although an example is shown in which the retracted position and the detection position are switched by moving the holder 6 in the vertical direction (Y-direction) orthogonal to the optical axis direction, the present invention is not limited to this. For example, like in the radiation phase contrast imaging apparatus 600 shown in FIG. 12, it may be configured to switch between the retracted position (see the two-dot chain line) and the detection position by rotatably moving the holder 6 about the axis line of the optical axis direction as a rotation axis about the center 23 of the holder 6.

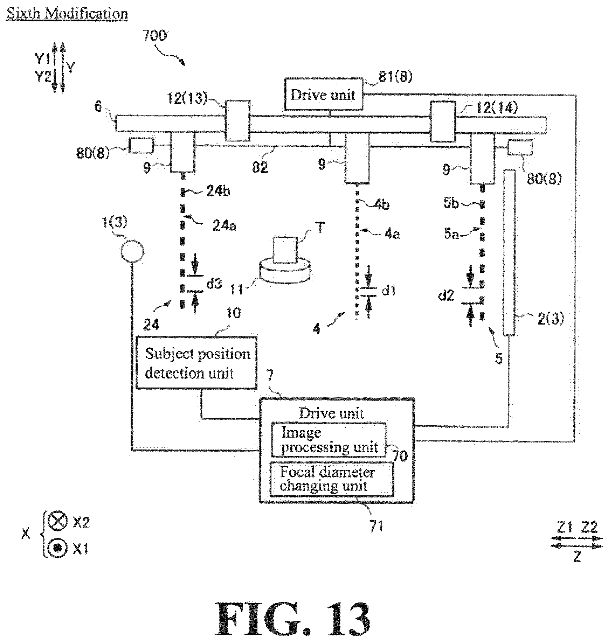

Further, in the aforementioned embodiment, although an example is shown in which the first grating 4 and the second grating 5 are provided as a plurality of gratings, the present invention is not limited to this. For example, like in the radiation phase contrast imaging apparatus 700 shown in FIG. 13, the third grating 24 may be provided between the X-ray source 1 and the first grating 4. The third grating 24 has a plurality of slits 24a and X-ray phase change portions 24b arranged at a predetermined period (pitch) d3 in the Y-direction. The slits 24a and the X-ray absorption portions 24b are each formed so as to extend linearly. Further, the slits 24a and the X-ray absorption portions 24b are each formed so as to extend in parallel with each other. Further, the third grating 24 is arranged between the X-ray source 1 and the first grating 4 and is configured to be irradiated by the X-rays from the X-ray source 1. The third grating 24 is configured to use the X-rays that have passed through each slit 24a as a line light source corresponding to the position of the each slits 24a. With this, the third grating 24 can enhance the coherence of the X-rays emitted from the X-ray source 1. With this, by the third grating 24, the coherence of the X-rays irradiated from the X-ray source can be enhanced. As a result, it is possible to form the self-image of the first grating 4 without depending on the focal length of the X-ray source 1, so that the freedom of selection of the X-ray source 1 can be improved.

Further in the aforementioned embodiment, although an example is shown in which the focal diameter of the X-ray source 1 is changed by changing the focus control of the X-ray source 1, the present invention is not limited to this. For example, it may be configured such that a plurality of X-ray sources different in focal diameters is provided and the focal diameter of the X-ray source 1 is changed by changing the X-ray source to be used.

Further in the aforementioned embodiment, although an example is shown in which the holder 6 is guided by the first guide member 13 and the second guide member 14, the present invention is not limited thereto. It may be configured to use two or more guide members. In this case, by providing one first guide member 13 having the second positioning portion 12b for positioning in the optical axis direction (Z-direction) and by providing two or more second guide members 14 not having the second positioning portion 12b, the optical axis direction of the holder 6 (Z-direction) can be determined.

Also, in the above embodiment, although an example is shown in which the holder 6 holds a plurality of gratings including the first grating 4 and the second grating 5, the present invention is not limited thereto. For example, it may be configured such that the image signal generation system 3 including the X-ray source 1 and the image signal detector 2 is held by a single holder 6.

## Claims

1. A radiation phase contrast imaging apparatus comprising:
an image signal generation system (3) including an X-ray source (1) and an image signal detector (2) for detecting an image signal based on X-rays irradiated from the X-ray source;
a plurality of gratings including a first grating (4) to which the X-rays from the X-ray source are irradiated and a second grating (5) arranged between the first grating and the image signal detector;
a holder (6) extending in an optical axis direction of the X-rays, the holder being configured to hold one of the plurality of gratings and the image signal generation system in a suspended manner; and
a position switching mechanism (8) configured to switch a relative position of the plurality of gratings with respect to the image signal generation system between a retracted position for capturing an absorption image without using the plurality of gratings and a detection position for capturing a phase contrast image using the plurality of gratings,
wherein either one of the plurality of gratings and the image signal generation system is held by the holder which is single in number.

2. The radiation phase contrast imaging apparatus as recited in claim 1, wherein the position switching mechanism includes a moving mechanism (80) configured to move the holder between the retracted position and the detection position by synchronously moving both ends of the holder.

3. The radiation phase contrast imaging apparatus as recited in claim 2, wherein
the moving mechanism is provided at each of both ends of the holder, and is configured to be driven by a single drive unit (81).

4. The radiation phase contrast imaging apparatus as recited in any of claims 1 to 3, further comprising a positioning portion (12) for determining a position of the holder in the detection position by coming into contact with the holder.

5. The radiation phase contrast imaging apparatus as recited in claim 4, wherein the positioning portion is configured to determine the position of the holder in a vertical direction orthogonal to the optical axis direction by coming into contact with the holder from below the holder.

6. The radiation phase contrast imaging apparatus as recited in claim 4 or 5, wherein
the positioning portion is configured to determine the position of the holder in the optical axis direction by coming into contact with the holder from the optical axis direction.

7. The radiation phase contrast imaging apparatus as recited in any of claims 4 to 6, further comprising a guide member having a hole part extending in a vertical direction orthogonal to the optical axis direction, wherein
the holder includes a protrusion (60) protruding in the hole part, and
the hole part is provided with a first positioning portion (12a) for determining the position of the holder in a vertical direction orthogonal to the optical axis direction and a second positioning portion (12b) for determining the position of the holder in the optical axis direction.

8. The radiation phase contrast imaging apparatus as recited in claim 7, wherein the first positioning portion is provided on an inner peripheral surface of a lower end portion of the hole part, and
the second positioning portion is provided on an inner peripheral surface of the hole part in the optical axis direction.

9. The radiation phase contrast imaging apparatus as recited in claim 7 or 8, wherein
the guide member is arranged so as to sandwich the holder from both sides in a horizontal direction orthogonal to the optical axis direction and configured to determine the position of the holder in the horizontal direction orthogonal to the optical axis direction.

10. The radiation phase contrast imaging apparatus as recited in any of claims 4 to 9, wherein
the position switching mechanism includes a contact part (80b) for raising and lowering the holder by coming into contact with the holder,
the position switching mechanism is provided below than the positioning portion in a vertically movable manner, and
the holder is configured to be supported by the positioning portion in the detection position in a state of being detached from the contact part of the position switching mechanism.

11. The radiation phase contrast imaging apparatus as recited in any of claims 1 to 10, further comprising a subject position detection unit (10) configured to detect a position of the subject, wherein
when the subject is placed in an area in which the plurality of gratings is arranged at the time of placing the holder from the retracted position to the detection position, and
the subject position detection unit is configured to prohibit an arrangement of the holder to the detection position.

12. The radiation phase contrast imaging apparatus as recited in any of claims 1 to 11, further comprising a movable subject stage (11) for placing a subject, the subject stage being arranged below the holder so as to face the holder.

13. The radiation phase contrast imaging apparatus as recited in any of claims 1 to 12, wherein
the holder is provided with a grating position adjustment mechanism (9), and the grating position adjustment mechanism is configured to adjust a relative position of the plurality of gratings between gratings when the holder is placed in the detection position from the retracted position.

14. The radiation phase contrast imaging apparatus as recited in any of claims 1 to 13, further comprising a focal diameter changing unit (71) configured to change a focal diameter of the X-ray source in conjunction with switching of the holder between the retracted position and the detection position.
